# EUROPEAN PATENT APPLICATION

(11) **EP 4 470 448 A1**
(43) Date of publication of application: **04.12.2024**
(21) Application number: 22923803.5
(22) Date of filing: 26.01.2022
(51) Int. Cl.: A61B 1/045

(54) **IMAGE-DETERMINING DEVICE, IMAGE-DETERMINING METHOD, AND RECORDING MEDIUM**

(71) Applicant: NEC Corporation, 108-8001 Tokyo (JP)
(72) Inventor: MARUGAME, Atsushi, Tokyo 108-8001 (JP)
(74) Representative: Betten & Resch
(86) International application number: PCT/JP2022/002920
(87) International publication number: WO 2023/144936

(57) **Abstract**

The acquisition means acquires an endoscopic image. The detection means detects a lesion candidate from the endoscopic image and output a lesion candidate image including the lesion candidate. The setting means sets a lesion area corresponding to the lesion candidate and a corresponding area corresponding to the lesion area, in the lesion candidate image. The blur determination means determines a blur of an image of the lesion area based on the image of the lesion area and an image of the corresponding area. The image determination means determines suitability of the lesion candidate image based on a determination result of the blur.

## Description

### TECHNICAL FIELD

The present disclosure relates to determination of images obtained by an endoscopic examination.

### BACKGROUND ART

In an endoscopic examination, a part including a lesion candidate in a cylindrical body tissue is photographed by irradiating light. Because of the optical characteristics of the lens, the range in which the details can be distinguished becomes narrow when the light is intensely irradiated. In the case of photographing in a travel direction, the lesion candidate part is blurred, and the image sometimes becomes a so-called rear focus image that is focused on a relatively dark part. In addition, when the lens is too close to the lesion candidate, the part of the lesion candidate is blurred, and as a result the image sometimes becomes the rear focus image. In such a case, it becomes difficult to distinguish the lesion candidate, so it becomes an unsuitable image which is difficult to diagnose. Patent Document 1 proposes to provide a medical imaging device and an endoscope device capable of efficiently evaluating the imaging signal input continuously.

### PRECEDING TECHNICAL REFERENCES

### PATENT DOCUMENT

Patent Document 1: Japanese Patent Application Laid-Open under No. JP 2019-162371

### SUMMARY

### PROBLEM TO BE SOLVED

However, according to Patent Document 1, it is not always possible to efficiently select the endoscopic image used for diagnosis.

It is an object of the present disclosure to provide an image determination device capable of determining whether or not the endoscopic image photographed by endoscopy can be used for diagnosis.

### MEANS FOR SOLVING THE PROBLEM

According to an example aspect of the present disclosure, there is provided an image determination device comprising:
an acquisition means configured to acquire an endoscopic image;
a detection means configured to detect a lesion candidate from the endoscopic image and output a lesion candidate image including the lesion candidate;
a setting means configured to set a lesion area corresponding to the lesion candidate and a corresponding area corresponding to the lesion area, in the lesion candidate image;
a blur determination means configured to determine a blur of an image of the lesion area based on the image of the lesion area and an image of the corresponding area; and
an image determination means configured to determine suitability of the lesion candidate image based on a determination result of the blur.

According to another example aspect of the present disclosure, there is provided an image determination method comprising:
acquiring an endoscopic image;
detecting a lesion candidate from the endoscopic image and output a lesion candidate image including the lesion candidate;
setting a lesion area corresponding to the lesion candidate and a corresponding area corresponding to the lesion area, in the lesion candidate image;
determining a blur of an image of the lesion area based on the image of the lesion area and an image of the corresponding area; and
determining suitability of the lesion candidate image based on a determination result of the blur.

According to still another example aspect of the present disclosure, there is provided a recording medium storing a program, the program causing a computer to execute processing of:
acquiring an endoscopic image;
detecting a lesion candidate from the endoscopic image and output a lesion candidate image including the lesion candidate;
setting a lesion area corresponding to the lesion candidate and a corresponding area corresponding to the lesion area, in the lesion candidate image;
determining a blur of an image of the lesion area based on the image of the lesion area and an image of the corresponding area; and
determining suitability of the lesion candidate image based on a determination result of the blur.

### EFFECT

According to the present disclosure, it is possible to determine whether or not the endoscopic image obtained by photographing a lesion candidate can be used for diagnosis.

### BRIEF DESCRIPTION OF THE DRAWINGS

[FIG. 1] FIG. 1 is a block diagram showing a schematic configuration of an endoscopic examination system.
[FIG. 2] FIG. 2 is a block diagram showing a hardware configuration of an image processing device.
[FIG. 3] FIG. 3 is a block diagram showing a functional configuration of the image processing device.
[FIG. 4] FIGS. 4A and 4B are diagrams showing an example of a corresponding area.
[FIG. 5] FIGS. 5A and 5B are diagrams showing a method of calculating power of a high frequency component.
[FIG. 6] FIG. 6 is a diagram showing an example of a determination result.
[FIG. 7] FIG. 7 is a diagram showing a display example of the determination result.
[FIG. 8] FIG. 8 is a flowchart of determination processing by an image determination device.
[FIG. 9] FIG. 9 is a block diagram showing a functional configuration of an image determination device of a second example embodiment.
[FIG. 10] FIG. 10 is a flowchart of processing by the image determination device of the second example embodiment.

### EXAMPLE EMBODIMENTS

Preferred example embodiments of the present disclosure will be described with reference to the accompanying drawings.

### <First example embodiment>

### [System configuration]

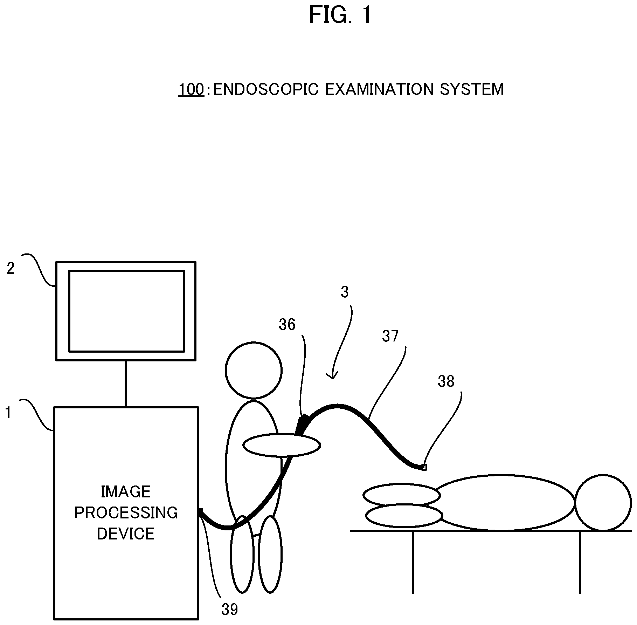

FIG. 1 shows a schematic configuration of an endoscopic examination system 100. The endoscopic examination system 100 detects a lesion-like part (hereinafter, referred to as a "lesion candidate"), and outputs an endoscopic image including the detected lesion candidate (hereinafter, referred to as a "lesion candidate image"), at the time of examination (including treatment) using the endoscope. The endoscopic examination system 100 determines whether or not the lesion candidate image can be used for diagnosing by a doctor or AI (Artificial Intelligence), and displays the result. This makes it possible to select the lesion candidate image that can be used for diagnosis, and diagnose the lesion candidate efficiently.

As shown in FIG. 1, the endoscopic examination system 100 mainly includes an image processing device 1, a display device 2, and an endoscope 3 connected to the image processing device 1.

The image processing device 1 acquires a moving image (i.e., a movie, hereinafter also referred to as an "endoscopic video Ic") captured by the endoscope 3 during the endoscopic examination from the endoscope 3 and displays display data for the check by the examiner of the endoscopic examination on the display device 2. Specifically, the image processing device 1 acquires a moving image of organs captured by the endoscope 3 as an endoscopic video Ic during the endoscopic examination. In addition, when the examiner finds a lesion during the endoscopic examination, he or she operates the endoscope 3 to input a photographing instruction of the lesion position. Based on the photographing instruction by the examiner, the image processing device 1 generates a lesion image capturing the lesion position. Specifically, the image processing device 1 generates the lesion image which is a still image, from the endoscopic video Ic which is a moving image, on the basis of the photographing instruction of the examiner.

The display device 2 is a display or the like for displaying images on the basis of the display signal supplied from the image processing device 1.

The endoscope 3 mainly includes an operation unit 36 used by the examiner to input instructions such as air supply, water supply, angle adjustment, and the photographing instruction, a shaft 37 having flexibility and inserted into an organ of a subject to be examined, a tip portion 38 with a built-in image-taking unit such as an ultra-compact imaging element, and a connection unit 39 for connection with the image processing device 1.

### [Hardware configuration]

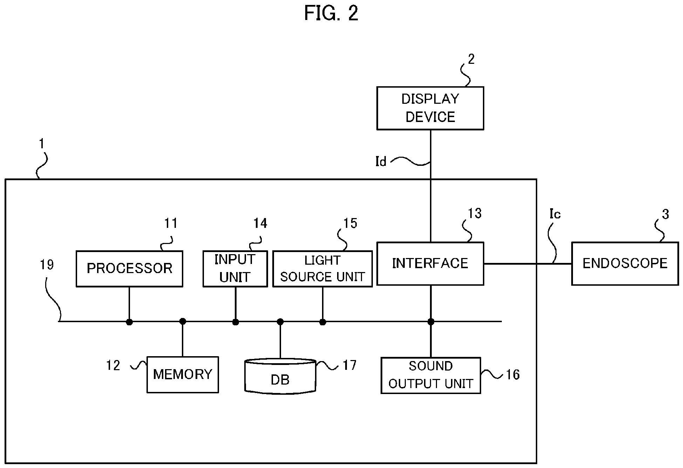

FIG. 2 shows a hardware configuration of the image processing device 1. The image processing device 1 mainly includes a processor 11, a memory 12, an interface 13, an input unit 14, a light source unit 15, a sound output unit 16, and a data base (hereinafter referred to as "DB") 17. These elements are connected with each other via a data bus 19.

The processor 11 executes predetermined processing by executing a program stored in the memory 12. The processor 11 is a processor such as a CPU (Central Processing Unit), a GPU (Graphics Processing Unit), and a TPU (Tensor Processing Unit). The processor 11 may be configured by multiple processors. The processor 11 is an example of a computer.

The memory 12 is configured by various volatile memories used as a working memory and non-volatile memories for storing information needed for the processing of the image processing device 1, such as a RAM (Random Access Memory) and a ROM (Read Only Memory). Incidentally, the memory 12 may include an external storage device such as a hard disk connected to or incorporated in the image processing device 1, and may include a storage medium such as a removable flash memory or a disk medium. The memory 12 stores a program for the image processing device 1 to execute processing in the present example embodiment.

Also, the memory 12 temporarily stores a series of endoscopic video Ic taken by the endoscope 3 in the endoscopic examination, based on the control of the processor 11. Further, the memory 12 temporarily stores the lesion images photographed in response to the photographing instructions by the examiner during the endoscopic examination. These images are stored in the memory 12 in association with, for example, subject identification information (e.g., the patient ID) and time stamp information, etc.

The interface 13 performs an interface operation between the image processing device 1 and the external devices. For example, the interface 13 supplies the display data Id generated by the processor 11 to the display device 2. Also, the interface 13 supplies the illumination light generated by the light source unit 15 to the endoscope 3. Also, the interface 13 supplies an electrical signal indicating the endoscopic video Ic supplied from the endoscope 3 to the processor 11. The interface 13 may be a communication interface such as a network adapter for wired or wireless communication with an external device, or may be a hardware interface compliant with a USB (Universal Serial Bus), SATA (Serial Advanced Technology Attachment), etc.

The input unit 14 generates an input signal based on the operation of the examiner. The input unit 14 is, for example, a button, a touch panel, a remote controller, a voice input device, or the like. The light source unit 15 generates the light to be delivered to the tip portion 38 of the endoscope 3. The light source unit 15 may also incorporate a pump or the like for delivering water or air to be supplied to the endoscope 3. The sound output unit 16 outputs the sound based on the control of the processor 11.

The DB 17 stores the endoscopic video acquired by past endoscopic examinations of the subject, and the lesion information. The lesion information includes lesion image and related information. The DB 17 may include an external storage device, such as a hard disk connected to or incorporated in the image processing device 1, and may include a storage medium, such as a removable flash memory. Instead of providing the DB 17 in the endoscopic examination system 100, the DB 17 may be provided in an external server or the like to acquire associated information from the server through communication.

### [Functional configuration]

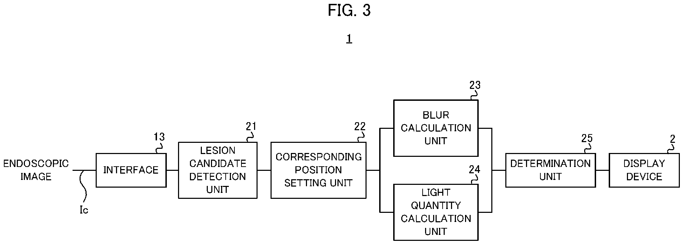

FIG. 3 is a block diagram showing a functional configuration of the image processing device 1. The image processing device 1 functionally includes the interface 13, a lesion candidate detection unit 21, a corresponding position setting unit 22, a blur calculation unit 23, a light quantity calculation unit 24, a determination unit 25, and the display device 2.

The image processing device 1 receives the endoscopic video Ic from the endoscope 3. The endoscopic video Ic is inputted to the interface 13. The interface 13 outputs the inputted endoscopic video Ic to the lesion candidate detection unit 21.

The lesion candidate detection unit 21 detects the lesion candidate from the endoscopic video Ic and generates the lesion candidate image including the detected lesion candidate. Here, the lesion candidate detection unit 21 may detect the lesion candidate included in the endoscopic video Ic and generate the lesion candidate image by using AI image analysis, or may generate the lesion candidate image based on the photographing instruction of the examiner during the endoscopic examination. The lesion candidate detection unit 21 outputs the generated lesion candidate image to the corresponding position setting unit 22.

The corresponding position setting unit 22 encloses the lesion candidate on the lesion candidate image with a rectangle or the like, and sets the area enclosed by the rectangle or the like as the lesion area. Further, the corresponding position setting unit 22 sets a corresponding area on the lesion candidate image. The corresponding area is set at a position corresponding to the lesion area on the lesion candidate image.

Here, a setting method of the corresponding area by the corresponding position setting unit 22 is explained as follows. Since the endoscope photographs the cylindrical body tissue, in many cases, an upper part of the endoscopic image corresponds to the front side and a lower part of the endoscopic image corresponds to the back side, or the upper part corresponds to the back side and the lower part corresponds to the front side. Also, when photographing while inserting or removing the endoscope, since the image has a depth, it is difficult to focus on the entire image or apply light equally. Therefore, the endoscopic image, where the image quality is good in the foreground but blurred in the background or the image quality is blurred in the foreground but good in the background, is generated.

Therefore, when there is the lesion area in the upper part of the lesion candidate image, the corresponding position setting unit 22 sets one or a plurality of corresponding areas in the lower part of the lesion candidate image. Further, when there is the lesion area in the lower part of the lesion candidate image, the corresponding position setting unit 22 sets one or a plurality of corresponding areas in the upper part of the lesion candidate image. Thus, the lesion candidate image set with the lesion area and the corresponding area is used for image determination as described below.

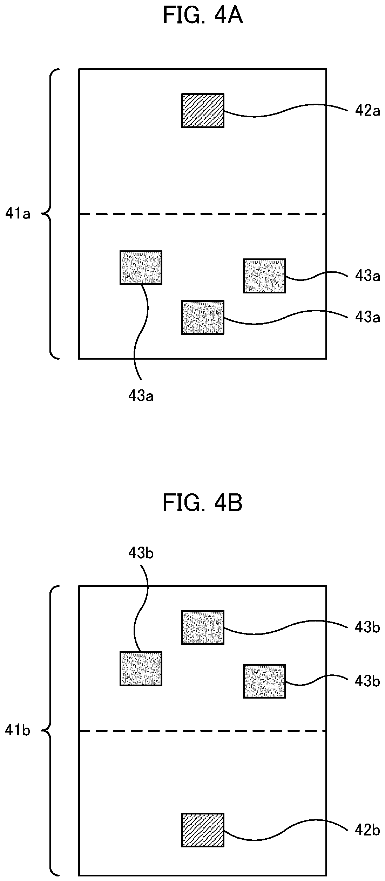

Specifically, FIG. 4 shows an example of a corresponding area set on a certain lesion candidate image. Incidentally, in FIG. 4A and FIG. 4B, the broken line in the center is described for convenience for dividing the image into the upper part and the lower part. In the example shown in FIG. 4A, there is a lesion area 42a (diagonally shaded rectangle) on the upper part of a lesion candidate image 41a. At this time, the corresponding position setting unit 22 sets one or a plurality of corresponding area 43a (gray rectangle) to the lower part of the lesion candidate image 41a. Also, in the example shown in FIG. 4B, there is a lesion area 42b (diagonally shaded rectangle) on the lower part of a lesion candidate image 41b. At this time, the corresponding position setting unit 22 sets one or a plurality of corresponding area 43b (gray rectangle) to the upper part of the lesion candidate image 41b. Incidentally, the corresponding position setting unit 22 may set the corresponding area at the position that is vertically symmetrical with the lesion area, or set one or a plurality of corresponding areas at the position shifted left and right from the position that is vertically symmetrical with the lesion area. Further, when photographing the inside of the tubular tissue, the corresponding position setting unit 22 considers the shape of the tubular and may set a plurality of corresponding areas on the arc.

The corresponding position setting unit 22 outputs the lesion candidate image, to which the lesion area and the corresponding area are set as described above, to the blur calculation unit 23 and the light quantity calculation unit 24.

The blur calculation unit 23 performs Fourier transform and calculates power of high frequency component, for each of the image of the lesion area and the image of the corresponding area set by the corresponding position setting unit 22. Then, the blur calculation unit 23 outputs the calculation result to the determination unit 25.

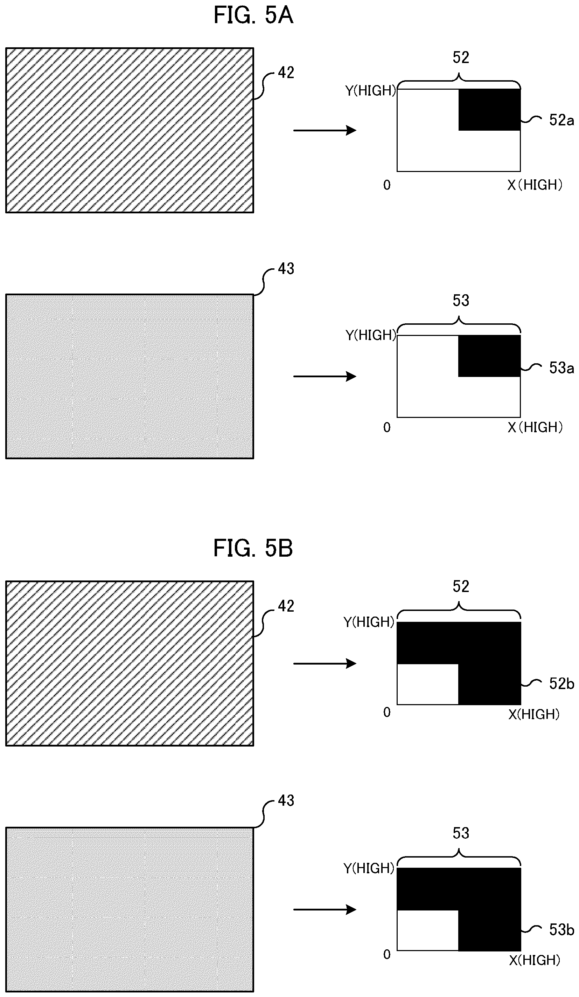

Here, a method of calculating the power of high frequency component by the blur calculation unit 23 is explained as follows. For a certain image data, a sharp image in focus will have a larger power of high frequency component, while a blurred image will have a smaller power of high frequency component. Therefore, it is possible to determine whether or not the image is in focus or blurred by calculating the power of high frequency component of the image. FIGS. 5A and 5B show a method of calculating the power of high frequency component by the blur calculation unit 23.

In FIG. 5A, a lesion area image 42 and a corresponding area image 43 are subjected to Fourier transform, and two-dimensional arrays 52 and 53 are obtained, respectively. Each element of the two-dimensional array contains the power of frequency component (a norm of a complex number). Further, the frequency becomes higher toward the right direction (X direction) and the upward direction (Y direction) in the two-dimensional arrays 52 and 53. Therefore, the blur calculation unit 23 sets the upper right area where the values of X and Y are large in the two-dimensional arrays 52 and 53 (black area) as the high frequency areas 52a and 53a, calculates the power of high frequency areas 52a and 53a, respectively, and outputs the calculation result to the determination unit 25. Instead, as in FIG. 5B, the blur calculation unit 23 may set the area where the value of either X or Y is large, i.e., the black area other than the lower left as the high frequency areas 52b and 53b, calculate the power of high frequency areas 52b and 53b, respectively, and output the calculation result to the determination unit 25.

The light quantity calculation unit 24 calculates the light quantity for each of the lesion area image and the corresponding area image set by the corresponding position setting unit 22, and outputs the calculation result to the determination unit 25. Specifically, the light quantity calculation unit 24 converts RGB values of the lesion area image, which is an RGB image, into HSV values. Then, light quantity calculation unit 24 outputs the obtained brightness value (V value) as the light quantity to the determination unit 25. The light quantity calculation unit 24 performs the same processing about the corresponding area image, and outputs the brightness value (V value) to the determination unit 25. The method of calculating the light quantity is not limited thereto, and for example, the light quantity may be calculated using another color space conversion method or the like.

The determination unit 25 determines whether or not the lesion candidate image is a suitable image that can be used for diagnosis, based on the result of the blur calculation unit 23 and the light quantity calculation unit 24. Specifically, first, the determination unit 25 performs power determination of the high frequency component based on the output of the blur calculation unit 23, and determines whether the lesion area image is in focus or blurred. Then, if the lesion area image is in focus, the determination unit 25 determines that the lesion candidate image is the suitable image. On the other hand, if the lesion area image is blurred, the determination unit 25 determines that the lesion candidate image is the unsuitable image. Furthermore, the determination unit 25 performs the light quantity (brightness) determination and determines the light and darkness of the lesion area image and the corresponding area image. Then, the determination unit 25 estimates the cause why the lesion candidate image is unsuitable based on the results of the power determination of high frequency component and the light quantity determination.

First, the power determination of high frequency component will be described in detail. The power determination of high frequency component refers to the determination whether or not the focus is correct for each of the lesion area image and the corresponding area image by the determination unit 25.

Specifically, the determination unit 25 compares the power of high frequency component of the lesion area image and the power of high frequency component of the corresponding area image calculated by the blur calculation unit 23 with a predetermined threshold TH1 which is determined in advance. The predetermined threshold TH1 is a value of the boundary as to whether or not the image is in focus. When the power of high frequency component is equal to or larger than the predetermined threshold TH1 about the lesion area image, the determination unit 25 determines it as "large", i.e. the image is in focus. When the power of high frequency component is smaller than the predetermined threshold TH1 about the lesion area image, the determination unit 25 determines it as "small", i.e. the image is blurred. Similarly, also for the corresponding area image, the determination unit 25 determines it as "large" when the power of high frequency component is equal to or larger than the predetermined threshold TH1, and as "small" when the power of high frequency component is smaller than the predetermined threshold TH1.

If the plurality of corresponding areas is set on the lesion candidate image, the determination unit 25 may determine using the one corresponding area that has the largest difference in the power of high frequency component from the lesion area among the plurality of correspondence areas.

When it is determined that the lesion area image is in focus by the power determination of high frequency component, the determination unit 25 outputs a result that the lesion candidate image is the suitable image that can be used for diagnosis. On the other hand, when it is determined that the lesion area image is blurred, the determination unit 25 outputs a result that the lesion candidate image is the unsuitable image that cannot be used for diagnosis.

Incidentally, the determination unit 25 may be provided with a predetermined threshold TH2 in addition to the predetermined threshold TH1, and determine whether or not the image is in focus. The predetermined threshold TH2 is a value of the boundary as to whether or not the image is blurred. For each of the lesion area image and the corresponding area image, the determination unit 25 determines it as "large" when the power of high frequency component is equal to or larger than the predetermined threshold TH1, and determines it as "small" when the power of high frequency component is equal to or smaller than the predetermined threshold TH2. Further, when the power of high frequency component of the lesion area image is smaller than the predetermined threshold TH1 and larger than the predetermined threshold TH2, the determination unit 25 may determine it as "large" or let the examiner select whether or not the image is used for diagnosis.

Next, the light quantity (brightness) determination will be described in detail. The light quantity (brightness) determination is that the determination unit 25 determines the brightness and darkness for each image of the lesion area image and the corresponding area image.

Specifically, the determination unit 25 compares the light quantity of the lesion area image and the light quantity of the corresponding area image calculated by the light quantity calculation unit 24, with a predetermined threshold TH3 determined in advance. Then, when the light quantity of the lesion area image is equal to or larger than the predetermined threshold TH3, the determination unit 25 determines it as "large", i.e., the light quantity is large. On the other hand, when the light quantity of the lesion area image is smaller than the predetermined threshold TH3, the determination unit 25 determines it as "small", i.e., the light quantity is small. Similarly, for the corresponding area image, when the light quantity is equal to or larger than the predetermined threshold TH3, the determination unit 25 determines it as "large", and when smaller than the predetermined threshold TH3, the determination unit 25 determines it as "small".

The determination unit 25 may be provided with a predetermined threshold TH4 in addition to the predetermined threshold TH3, and determine the brightness and darkness. For example, the predetermined threshold TH3 is a value of the boundary as to whether or not the light quantity is large, and the predetermined threshold TH4 is a value of the boundary as to whether or not the light quantity is small. Then, for each of the lesion area image and the corresponding area image, the determination unit 25 determines it as "large" when the light quantity is equal to or larger than the predetermined threshold TH3, and the determination unit 25 determines it as "small" when the light quantity is equal to or smaller than the predetermined threshold TH4. Further, when the light quantity is smaller than the predetermined threshold TH3 and larger than the predetermined threshold TH4, the determination unit 25 determines that the light quantity is suitable.

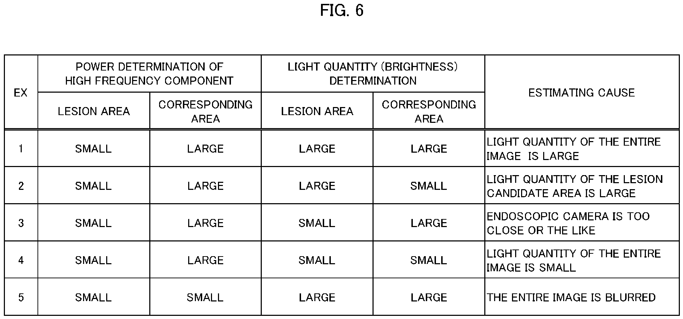

FIG. 6 shows an example of a determination result when the lesion candidate image is determined to be unsuitable by the determination unit 25. As shown, the determination unit 25 outputs the determination result including "the power determination of high frequency component", "the light quantity (brightness) determination", and "the estimating the cause".

"The power determination of high frequency component" indicates the determination result of the power of high frequency component of the lesion area and the corresponding area. The determination result "large" indicates that the image is in focus, and the determination result "small" indicates that the image is blurred. In the example of FIG. 6, the lesion area image is blurred in all cases.

"The light quantity (brightness) determination" indicates the determination result of the light quantity (brightness) of the lesion area and the corresponding area. The determination result "large" indicates that the light quantity is large, and the determination result "small" indicates that the light quantity is small.

"The estimating the cause" indicates the cause that the lesion candidate image is the unsuitable image. The determination unit 25 estimates the cause that the lesion area image is not in focus, based on the result of "the power determination of high frequency component" and "the light quantity (brightness) determination". For example, in example 1 of FIG. 6, since both the light quantity of the lesion area and the corresponding area are large, the determination unit 25 estimates the cause of the unsuitable lesion candidate image as "light quantity of the entire lesion candidate image is large". In example 2, since the light quantity of the lesion area is large, the determination unit 25 estimates the cause of the unsuitable lesion candidate image as "light quantity of the lesion candidate area is large". In example 3, since the light quantity of the corresponding area is large, the determination unit 25 estimates the cause of the unsuitable lesion candidate image as "endoscopic camera is too close or the like". In example 4, since the light quantity of both the lesion area and the corresponding area are small, the determination unit 25 estimates the cause of the unsuitable lesion candidate image as "light quantity of the entire lesion candidate image is small". In example 5, since the light quantity of both the lesion area and the corresponding area are large and the image is blurred in both the lesion area and the corresponding area, the determination unit 25 estimates the cause of the unsuitable lesion candidate image as "the entire lesion candidate image is blurred".

The determination unit 25 performs the determination as described above, and outputs the determination result to the display device 2. The display device 2 displays the determination result input from the determination unit 25.

### [Display example]

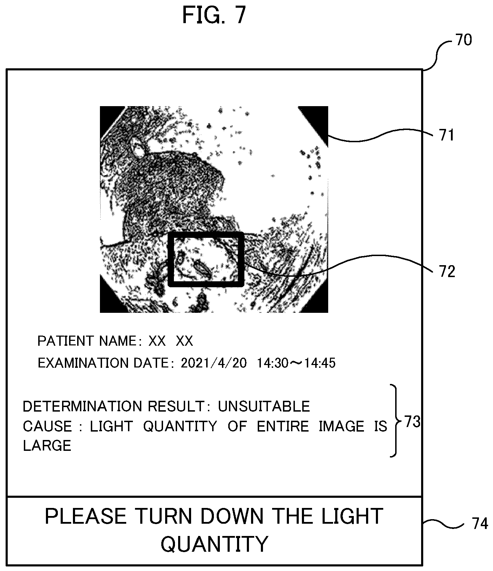

Next, a display example by the display device 2 will be described. FIG. 7 is an example of a display by the display device 2. In the example of FIG. 7, a lesion candidate image 71, a lesion area 72, basic information such as a patient name and examination date, related information 73 representing the determination result of the image and the cause, a message 74 prompting the adjustment of the light quantity are displayed in the display area 70 of the display device 2.

The lesion candidate image 71 is the endoscopic image of the organ site where the lesion candidate is detected. The lesion area 72 shows the lesion candidate included in the lesion candidate image 71 in a manner surrounded by a rectangle. The related information 73 is the determination result of whether or not the lesion candidate image 71 is the suitable image that can be used for diagnosis. If it is determined that the lesion candidate image 71 is the unsuitable image, the cause of the unsuitable image is also indicated in the related information 73. Further, the message 74 is the message prompting actions such as light quantity adjustment, and the message 74 is displayed when the lesion candidate image 71 is determined to be unsuitable. As such, in the present example embodiment, it is possible to easily grasp whether or not the lesion candidate image is an image suitable for diagnosis by viewing the related information 73 and the message 74.

### [Determination processing]

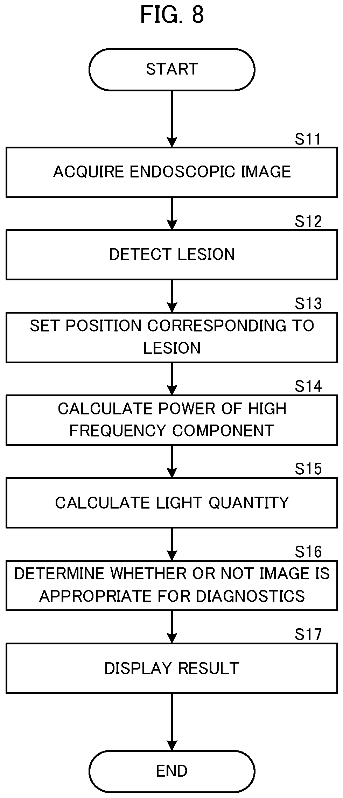

Next, determination processing for performing the above-mentioned determination will be described. FIG. 8 is a flowchart of the determination processing performed by the image processing device 1. This processing is realized by the processor 11 shown in FIG. 2, which executes a pre-prepared program and operates as each element shown in FIG. 3.

First, the interface 13 acquires the endoscopic video Ic (step S11). Next, the lesion candidate detection unit 21 detects the lesion candidate from the endoscopic video Ic and generates the lesion candidate images (step S12). Next, the corresponding position setting unit 22 sets the lesion area including the lesion candidate for the lesion candidate image, and sets the corresponding area for the lesion area (step S13). Incidentally, when there is the lesion area in the upper part of the lesion candidate image, the corresponding area is set in the lower part of the lesion candidate image, and when there is the lesion area in the lower part of the lesion candidate image, the corresponding area is set in the upper part of the lesion candidate image.

Next, the blur calculation unit 23 performs Fourier transform and calculates the power of high frequency component, for each of the lesion area image and the corresponding area image (step S14). Further, the light quantity calculation unit 24 converts RGB values of the lesion area image and the corresponding area image into HSV values, and calculates the respective light quantity (V value) (step S15). Then, the determination unit 25 determines whether or not the images are in focus by comparing the power of high frequency component of the lesion area image and the power of high frequency component of the corresponding area image with the predetermined threshold TH1. Further, the determination unit 25 determines the light and darkness of the images by comparing the light quantity of the lesion area image and the light quantity of the corresponding area image with the predetermined threshold TH3. Then, the determination unit 25 determines whether or not the lesion candidate image is the suitable image for diagnosis based on the above-described determination result (step S16). Incidentally, the determination unit 25 estimates the cause when the lesion candidate image is not the suitable image for diagnosis.

Then, the display device 2 displays the determination result of the determination unit 25 (step S 17). Thus, the display as shown in FIG. 7 is performed. Incidentally, step S15 may be performed prior to step S14, or may be performed simultaneously with step S15.

As described above, in this example embodiment, the endoscopic examination system 100 can select the image in which the lesion candidate is in focus from among the captured endoscopic images. This allows the doctor or AI to perform image diagnosis efficiently. In addition, even when the lesion candidate is blurred and cannot be used for image diagnosis, it is possible to urge the improvement of the setting by displaying the cause.

### <Second example embodiment>

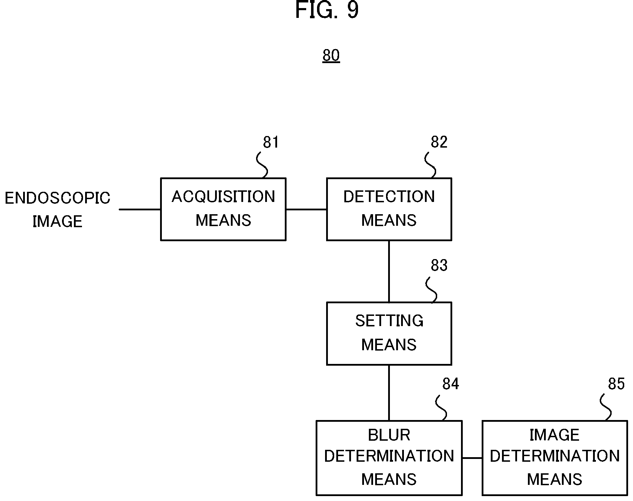

FIG. 9 is a block diagram showing a functional configuration of an image determination device according to the second example embodiment. The image determination device 80 includes an acquisition means 81, a detection means 82, a setting means 83, a blur determination means 84, and an image determination means 85.

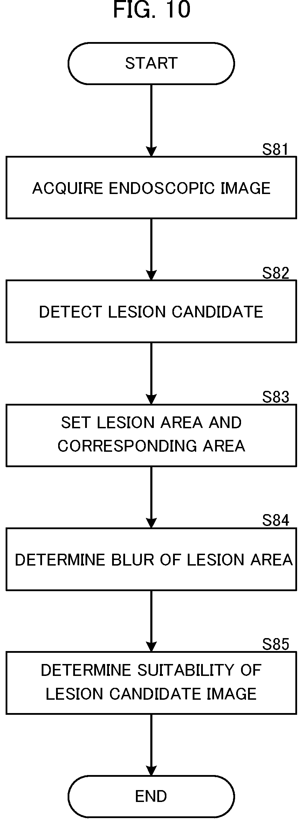

FIG. 10 is a flowchart of processing by the image determination device of the second example embodiment. The acquisition means 81 acquires an endoscopic image (step S81). The detection means 82 detects a lesion candidate from the endoscopic image and output a lesion candidate image including the lesion candidate (step S82). The setting means 83 sets a lesion area corresponding to the lesion candidate and a corresponding area corresponding to the lesion area, in the lesion candidate image (step S83). The blur determination means 84 determines a blur of an image of the lesion area based on the image of the lesion area and an image of the corresponding area (step S84). The image determination means 85 determines suitability of the lesion candidate image based on a determination result of the blur (step S85).

According to the image determination device 80 of the second example embodiment, it is possible to determine whether or not the endoscopic image with the lesion candidate can be used for diagnosis.

A part or all of the above example embodiments may also be described as in the following supplementary notes, but are not limited to:

### (Supplementary note 1)

An image determination device comprising:
an acquisition means configured to acquire an endoscopic image;
a detection means configured to detect a lesion candidate from the endoscopic image and output a lesion candidate image including the lesion candidate;
a setting means configured to set a lesion area corresponding to the lesion candidate and a corresponding area corresponding to the lesion area, in the lesion candidate image;
a blur determination means configured to determine a blur of an image of the lesion area based on the image of the lesion area and an image of the corresponding area; and
an image determination means configured to determine suitability of the lesion candidate image based on a determination result of the blur.

### (Supplementary note 2)

The image determination device according to Supplementary note 1, wherein the blur determination means determines the blur of the image of the lesion area based on power of high frequency component for each of the image of the lesion area and the image of corresponding area.

### (Supplementary note 3)

The image determination device according to Supplementary note 2, wherein the setting means sets one or more corresponding areas in a lower part of the lesion candidate image when there is the lesion candidate in an upper part of the lesion candidate image, and sets one or more corresponding areas in the upper part of the lesion candidate image when there is the lesion candidate in the lower part of the lesion candidate image.

### (Supplementary note 4)

The image determination device according to Supplementary note 3, wherein the blur determination means determines the blur of the image of the lesion area using a corresponding area that has a largest difference in the power of high frequency component between the lesion area and the corresponding area when there is a plurality of the corresponding area.

### (Supplementary note 5)

The image determination device according to any one of Supplementary notes 1 to 4, further comprising a light quantity calculation means configured to calculate light quantity of the image of the lesion area and the image of the corresponding area,
wherein the image determination means determines cause why the lesion candidate image is unsuitable based on the determination result of the blur and a determination result of the light quantity.

### (Supplementary note 6)

The image determination device according to Supplementary note 5, further comprising a display means configured to display an unsuitable lesion candidate image and a message indicating the cause why the lesion candidate image is unsuitable.

### (Supplementary note 7)

An image determination method comprising:
acquiring an endoscopic image;
detecting a lesion candidate from the endoscopic image and output a lesion candidate image including the lesion candidate;
setting a lesion area corresponding to the lesion candidate and a corresponding area corresponding to the lesion area, in the lesion candidate image;
determining a blur of an image of the lesion area based on the image of the lesion area and an image of the corresponding area; and
determining suitability of the lesion candidate image based on a determination result of the blur.

### (Supplementary note 8)

A recording medium storing a program, the program causing a computer to execute processing of:
acquiring an endoscopic image;
detecting a lesion candidate from the endoscopic image and output a lesion candidate image including the lesion candidate;
setting a lesion area corresponding to the lesion candidate and a corresponding area corresponding to the lesion area, in the lesion candidate image;
determining a blur of an image of the lesion area based on the image of the lesion area and an image of the corresponding area; and
determining suitability of the lesion candidate image based on a determination result of the blur.

While the present disclosure has been described with reference to the example embodiments and examples, the present disclosure is not limited to the above example embodiments and examples. Various changes which can be understood by those skilled in the art within the scope of the present disclosure can be made in the configuration and details of the present disclosure.

### DESCRIPTION OF SYMBOLS

1 Image processing device
2 Display device
3 Endoscope
11 Processor
12 Memory
13 Interface
17 Database (DB)
21 Lesion candidate detection unit
22 Corresponding position setting unit
23 Blur calculation unit
24 Light quantity calculation unit
25 Determination unit
100 Endoscopic examination system

## Claims

1. An image determination device comprising:
an acquisition means configured to acquire an endoscopic image;
a detection means configured to detect a lesion candidate from the endoscopic image and output a lesion candidate image including the lesion candidate;
a setting means configured to set a lesion area corresponding to the lesion candidate and a corresponding area corresponding to the lesion area, in the lesion candidate image;
a blur determination means configured to determine a blur of an image of the lesion area based on the image of the lesion area and an image of the corresponding area; and
an image determination means configured to determine suitability of the lesion candidate image based on a determination result of the blur.

2. The image determination device according to claim 1, wherein the blur determination means determines the blur of the image of the lesion area based on power of high frequency component for each of the image of the lesion area and the image of corresponding area.

3. The image determination device according to claim 2, wherein the setting means sets one or more corresponding areas in a lower part of the lesion candidate image when there is the lesion candidate in an upper part of the lesion candidate image, and sets one or more corresponding areas in the upper part of the lesion candidate image when there is the lesion candidate in the lower part of the lesion candidate image.

4. The image determination device according to claim 3, wherein the blur determination means determines the blur of the image of the lesion area using a corresponding area that has a largest difference in the power of high frequency component between the lesion area and the corresponding area when there is a plurality of the corresponding area.

5. The image determination device according to any one of claims 1 to 4, further comprising a light quantity calculation means configured to calculate light quantity of the image of the lesion area and the image of the corresponding area,
wherein the image determination means determines cause why the lesion candidate image is unsuitable based on the determination result of the blur and a determination result of the light quantity.

6. The image determination device according to claim 5, further comprising a display means configured to display an unsuitable lesion candidate image and a message indicating the cause why the lesion candidate image is unsuitable.

7. An image determination method comprising:
acquiring an endoscopic image;
detecting a lesion candidate from the endoscopic image and output a lesion candidate image including the lesion candidate;
setting a lesion area corresponding to the lesion candidate and a corresponding area corresponding to the lesion area, in the lesion candidate image;
determining a blur of an image of the lesion area based on the image of the lesion area and an image of the corresponding area; and
determining suitability of the lesion candidate image based on a determination result of the blur.

8. A recording medium storing a program, the program causing a computer to execute processing of:
acquiring an endoscopic image;
detecting a lesion candidate from the endoscopic image and output a lesion candidate image including the lesion candidate;
setting a lesion area corresponding to the lesion candidate and a corresponding area corresponding to the lesion area, in the lesion candidate image;
determining a blur of an image of the lesion area based on the image of the lesion area and an image of the corresponding area; and
determining suitability of the lesion candidate image based on a determination result of the blur.
